(19) Europäisches Patentamt
European Patent Office
Office européen des brevets

(11) Publication number: **0 274 863 B1**

(12) # EUROPEAN PATENT SPECIFICATION

(45) Date of publication of patent specification: **15.06.94**

(51) Int. Cl.[5]: **A61L 15/16**, A61F 13/02

(21) Application number: **87310744.5**

(22) Date of filing: **07.12.87**

(54) **Moisture-vapor-permeable dressing.**

(30) Priority: **09.01.87 US 2024**

(43) Date of publication of application:
**20.07.88 Bulletin 88/29**

(45) Publication of the grant of the patent:
**15.06.94 Bulletin 94/24**

(84) Designated Contracting States:
**DE FR GB IT**

(56) References cited:
**EP-A- 0 177 329**
**EP-A- 0 184 910**
**FR-A- 2 143 564**

(73) Proprietor: **HERCON LABORATORIES CORPORATION**
**200 B Corporate Court**
**Middlesex Business Center**
**South Plainfield New Jersey 07080(US)**

(72) Inventor: **Shah, Kishore R.**
**568 Cabot Hill Road**
**Bridgewater New Jersey 08807(US)**
Inventor: **Kydonieus, Agis**
**17 Savage Road**
**Kendall Park New Jersey 08824(US)**
Inventor: **Apostolopoulos, Dimitrios**
**122A Cedar Lane**
**Highland Park New Jersey 08904(US)**

(74) Representative: **Armitage, Ian Michael et al**
**MEWBURN ELLIS**
**York House**
**23 Kingsway**
**London WC2B 6HP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates broadly to articles of manufacture for administration of topical medicaments to human skin (as opposed to systemically active drugs) in a controlled release manner The articles are moisture-vapor-permeable and oxygen-permeable but are impermeable to water in the liquid phase. The articles consist essentially of:

(i) a polymeric backing which in many instances is elastomeric;

(ii) adhering to one surface of the polymeric backing, a medication reservoir which is a mixture of a polyvinyl chloride polymer, a polymeric plasticizer and a topical medicament; and

(iii) adhering to the medication reservoir on a surface opposite to backing layer is a pressure sensitive adhesive, Examples of topical medicaments useful in the practice of our invention are tolnaftate, chlorhexidine, chlorhexidine esters, chlorhexidine salts, hydrocortisone, benzalkonium salts, benzethonium salts and polyvinyl pyrrolidoneiodine complexes.

The terms "elastomeric" and "elastomer" are herein intended to cover polymers having reasonable degrees of elasticity; however, such "elastomer polymers" are to be expected to have finite yields. Thus, for example, an "elastomer" is a polymer herein intended to have an elongation greater than 150% under applied stress.

GB 1361289 describes a bandage for administering systemically active drugs. The bandage has three layers, a backing layer which is impervious to the drug, a reservoir usually in microcapsule form containing the drug and a pressure resistive adhesive.

The present invention provides an article of manufacture which offers enhanced delivery capability of a topical medicament to human skin in a controlled release manner directly from a plasticized vinyl resin layer (preferably a "plastisol") in which the topical medicament is incorporated in required high concentrations.

This invention enables the administration of topical medicaments such as tolnaftate, chlorhexidine, chlorhexidine esters, chlorhexidine salts, hydrocortisone, benzalkonium salts, benzethonium salts and iodine to be achieved through an article of manufacture, e.g., a controlled release delivery device requiring contact over the entire affected area of a patient tissue.

The present invention is set out in claim 1.

### Brief Description of the Drawings

Figure 1 is a cross-sectional view of a preferred topical medicament delivery device in accordance with this invention.

Figure 2 is another cross-sectional view of a second preferred topical medicament delivery device in accordance with this invention.

Figure 3 is a graph of stress relaxation versus time for various polyvinyl chloride, polyurethane and polyurethane-polyvinyl chloride backing films.

Figure 4 is a graph showing the fraction of release (weight percentages) versus time in hours for the release of hydrocortisone from a dressing as illustrated in Figure 2.

Figure 5 is the infrared spectrum of ADMEX ® 760 as used in Examples II-XII.

Figure 6 is the infrared spectrum of the polyester-polyurethane polymer used as the backing material in Examples II-XII, infra and indicated by reference numerals 17 in Figure 1 and 25 in Figure 2, supra.

The invention is described herein with respect to preferred embodiments including topical medicament delivery devices containing various topical medicaments including but not limited to:

(i) benzethonium chloride having the structure:

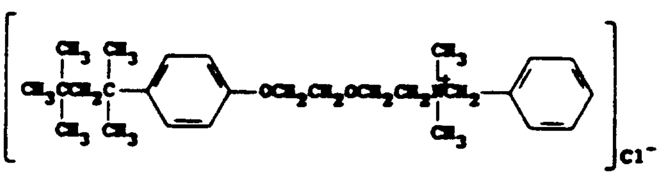

(benzyldimethyl(2-(2-(p-1,1,3,3-etetramethylbutyl-phenoxy)ethoxy)-ethylammonium chloride);

(ii) benzalkonium chloride having the structure:

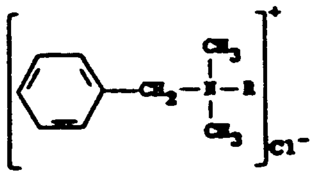

wherein R represents $C_8H_{17}$ up to $C_{18}H_{37}$ alkyl (defined according to U.S. Letters Patent 2,157,047);

(iii) hydrocortisone having the structure:

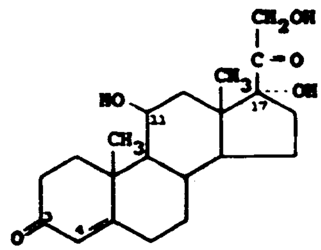

(4-pregnene-11 beta, 17 alpha, 21-triol-3,20-dione);

(iv) tolnaftate having the structure:

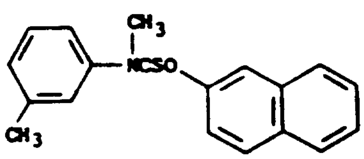

;

and

(v) chlorhexidine diacetate hydrate having the structure:

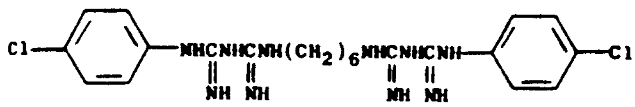

It will be understood that other embodiments may be employed within the spirit and scope of this invention. Thus, the foregoing examples are also intended to include the genuses, to wit:

(i) chlorhexidine, per se;

(ii) chlorhexidine esters;

(iii) chlorhexidine salts;

(iv) benzalkonium salts; and

(v) benzethonium salts.

In addition, another example of a topical medicament is iodine delivered through the substance polyvinylpyrrolidone-iodine complex.

In Figure 1 and in Figure 2 a cross-section of two embodiments of the article of our invention are set forth. In Figure 1, the backing layer is indicated by reference numeral 10. The topical medicament reservoir is indicated by reference numeral 11. The pressure-sensitive adhesive is indicated by reference numeral 12, Optionally, prior to use, a peelable layer 13 is removably adhered to the adhesive layer 12. The outer surface of the backing layer is indicated by reference numeral 17. The surface of the backing layer adhered

to the reservoir layer is indicated by reference numeral 16. The surface of the reservoir layer adhered to the pressure-sensitive adhesive layer is indicated by reference numeral 15. The surface of the pressure-sensitive adhesive layer adhered to the peelable backing layer is indicated by reference numeral 14. The surface of the peelable backing layer away from the adhesive layer is indicated by reference numeral 18.

Referring to Figure 2, the backing material lamina is indicated by reference numeral 20 with an outer surface 25 and an inner surface 23 to which is continuously adhered a medication reservoir lamina 21 having an outer surface 24 to which is adhered a pressure-sensitive material 22 having an outer surface 26.

The polymeric backing material lamina indicated by reference numeral 10 in Figure 1 and reference numeral 20 in Figure 2 has a thickness of between 0.5 and 1.5 mils (between 0.0005 inches ($1.3x10^{-5}$m) and 0.0015 inches ($3.8x10^{-4}$m)) and is composed of a mixture of polyurethane and polyvinyl chloride; from 5 up to 20% by weight of polyvinyl chloride and from 95 down to 80% by weight of polyurethane is preferred. Such films possess the required combination of breathability (oxygen and moisture vapor permeability) and handling and wear characteristics.

The over-all range of thickness of the article of our invention including backing layer, medication reservoir, and, pressure-sensitive adhesive may vary from 2.0 mils ($5x10^{-5}$m) up to about 6.0 mils ($1.5x10^{-4}$m) with a preferable maximum thickness of 4 mils ($1x10^{-4}$m).

Tests concerning the oxygen and water vapor permeability of the films, particularly the backing layer of our invention are set forth, infra.

The medication reservoir is comprised of a plasticized polyvinyl chloride resin (polyvinyl chloride in admixture with a non-migratory polymeric plasticizer), preferably, a "plastisol", containing the required concentration of the desired topical medication, Examples of topical medication used in the polyvinyl chloride resin are as follows:

(i) benzethonium chloride;
(ii) benalkonium chloride;
(iii) hydrocortisone;
(iv) tolnaftate;
(v) chlorhexidine; and
(vi) polyvinylpyrrolidone-iodine complex.

When using tolnaftate, the percent of tolnaftate in the polyvinyl chloride resin may vary from 0.5% up to 3.0%. When using chlorhexidine diacetate hydrate, for example, from 2 up to 12% by weight of the chlorhexidine diacetate hydrate may be used based upon the total weight of the medication reservoir. When using hydrocortisone, from 0.25% up to 3.0% by weight of the hydrocortisone based on the total weight of medication reservoir may be used. When using benzalkonium chloride, from 0.1% up to 1.0% by weight of the benzalkonium chloride may be used based upon the total weight of the medication reservoir. When using benzethonium chloride, from 0.1% up to 1% by weight of the benzethonium chloride based upon the total weight of the medication reservoir may be used. Preferred concentrations of topical medicament in the medication reservoir are set forth in the examples provided infra.

Thus, when using polyvinylpyrrolidone-iodine complex, between 7% and 11% by weight of the weight of medication reservoir may be used.

As stated supra, the moisture-vapor-permeable and oxygen-permeable dressing for use in supplying a topical medicament to human skin in a controlled release manner is to be impermeable to water in a liquid phase. Accordingly, the article of our invention must have a moisture-vapor-permeability of at least 25 g/square meters/24 hours at 25 °C and at 75% relative humidity; preferably, at least 40 g/square meter/24 hours at 25 °C and at 75% relative humidity. Accordingly, the polymeric backing material lamina of our invention can be a material other than the preferred polyurethane-polyvinyl chloride backing and can, in fact, be any of the backing materials set forth at column 5 and column 6 of USA Reissue Patent 31,886 issued on May 14, 1985. These include copolymers produced by copolymerizing in an inert atmosphere an alkoxy alkyl acrylate or methacrylate with a different alkoxy acrylate or methacrylate.

The medication reservoir as stated supra consists essentially of polyvinyl chloride and a polymeric plasticizer. The plasticized PVC layer contains from 20 up to 70% by weight of a polyvinyl chloride resin which consists essentially of a vinyl chloride polymer containing, predominantly or completely repeating vinyl chloride monomeric units and in an amount of less than 10% other repeating vinyl units, e.g., repeating vinyl acetate units; from 20% up to 70% by weight of the composition of one or more polymeric plasticizers; and from 0.25% up to 15% by weight of the total composition of a topical medicament, such as tolnaftate, chlorhexidine, chlorhexidine esters, chlorhexidine salts, hydrocortisone, benzalkonium salts, benzethonium salts or a polyvinylpyrrolidone-iodine complex or mixtures of the foregoing as exemplified hereinafter.

It has now been found that the release rate of active topical medicaments which is uniformly dispersed in the plasticized PVC layer is dependent upon the proportion of PVC resin in the composition. Thus, for example, the release follows the curve for hydrocortisone according to Figure 4.

The reservoir layers of this invention are relatively weak insofar as their tensile strengths are concerned and usually less than 1500 psi (highly flexible and soft materials). In contrast, the flexible vinyl films of commerce are considerably stronger materials exhibiting tensile strengths of the order of from 1500 psi ($7.2x10^{4}$ N/m$^2$) up to 5000 psi ($2.4x10^5$ N/m$^2$). The polyvinyl chloride resin content of the commercially useful and available flexible vinyl films range from 50 to 75%. Such materials do not have adequate topical medicament permeating characteristics to serve as suitable topical medicament delivery reservoirs. For example, a film for delivery of tolnaftate contains 43% polyvinyl chloride, 1% tolnaftate and 56% ADMEX ®, a polymeric plasticizer, A film containing chlorhexidine diacetate hydrate contains 41% polyvinyl chloride, 4% chlorhexidine diacetate hydrate and 55% ADMEX ®. The film containing hydrocortisone contains 42.5% polyvinyl chloride, 0.5% hydrocortisone and 57% ADMEX $^R$ . The film containing benzalkonium chloride contains 48.8% polyvinyl chloride, 0.2% benzalkonium chloride and 51% ADMEX ® as is the case with the film containing benzethonium chloride. This will be apparent from a reading of the examples set forth infra.

The topical medicament supplying devices of our invention comprises a plasticized polyvinyl chloride resin layer and a topical medicament agent substantially uniformly dispersed in the PVC layer which may be referred to as a reservoir for the topical medicament. The polyvinyl chloride reservoir in the present invention is prepared from a polyvinyl chloride resin and a primary polymeric plasticizer for the resin.

Preferably, the polyvinyl chloride resin employed in the preferable practice of the present invention is that which is specifically used in preparing PVC plastisols, namely PVC resins which are made by the well known emulsion polymerization process, which are hard spheres of particle size between 0.05 and 20 microns, such as between 1 and 20 microns, between 1 and 5 microns or between 0.05 and 1 micron, and which do not have the ability to absorb plasticizers to any great extent. Instead, the plasticizer wets the resin particles at room temperature and only then very slowly penetrates and solvates the resin, These PVC resins when mixed with the polymeric plasticizers such as a mixture of 30% polymeric plasticizer and 70% PVC resin, give a flowable liquid known as a "plastisol" which can then be fused at, for example, approximately 300 °F for approximately 60 seconds to provide a solid polymer layer. The PVC resin employed in the present invention is in contrast to the general purpose PVC resins which are produced by suspension or bulk polymerization and which are used in calendering and extrusion processes, which are 50 to 200 microns, such as 100 to 150 microns in diameter, and are like sponges. Thus, the general purpose resins are capable of absorbing large amounts of plasticizers so that even a 50% ADMEX ® and 50% PVC resin would result in a non-flowing solid material, The molecular weight of the PVC resins employed in the present invention preferably is a weight average molecular weight between 80,000 and 250,000, such as a weight average molecular weight of 123,000, A suitable polyvinyl chloride resin is one sold by Occidental Chemical Company under the designation FPC 6338 containing about 96% vinyl chloride monomer units and about 4% vinyl acetate monomer units, Thus, the polvinyl chloride resin can be a copolymer containing preferably at least 90% by weight vinyl chloride monomer units, such as a copolymer based on vinyl chloride and vinyl acetate.

The polyvinyl chloride resin generally is present in the layer in an amount of from 10 up to 50% weight percent, preferably 20 to 50 weight percent based on the total weight of the plasticized PVC composition.

The primary polymeric plasticizer which is employed in the present invention can be polymeric adipate plasticizers, which are polymers of adipic acid with a monomer, such as propylene glycol and, for example, can be obtained under the designation Drapex 334F from Witco Chemical Corporation, or any other known polymeric plasticizer for PVC which is biologically acceptable.

Thus, other examples of polyester adipates, glutarates and sebacates are;

polyester adipate P-644;
polyester glutarate P-530;
polyester glutarate P-540;
polyester glutarate P-550;
polyester glutarate P-7035;
polyester glutarate P-7035M;
polyester glutarate P-7046;
polyester glutarate P-7092; and
polyester sebacate P-1070

manufactured by the C.P. Hall Company, 7300 S. Central Avenue, Chicago, Illinois 60638. Other preferred polymeric plasticizers are those which are known as "adipate" plasticizers, for example, ADMEX ® 760 which is a high molecular weight linear adipate plasticizer manufactured by the Sherex Division of Nuodex

Inc, In general, polyester plasticizers which are polyesters of (i) adipic acid, 1,4-terephthalic acid and/or 1,2-phthalic acid with (ii) polyethylene glycols, ethylene glycol or 1,3-propylene glycol having molecular weights in the range of 4000-10,000 are preferred.

The polymeric plasticizer generally is present in an amount of between 50 and 70% based on the total weight of plasticized PVC layer.

With reference to Figures 1 and 2, the blended plasticized PVC containing, for example, PVC, ADMEX ® and hydrocortisone is then coated at a rate of 36 ounces/$yd^2$ (1222g/$m^2$) on a backing and then fused into solid plasticized PVC layer 11. The backing as stated supra is a single layer of drug impermeable polymer composition, preferably the polyvinyl chloride-polyurethane mixture as set forth supra.

The thickness of the medication reservoir lamina 11 (in Figure 1) and 21 (in Figure 2) is preferably about 1 mil but may vary from 0.5 mils up to 1.5 mils (from 0.0005 inches ($1.3x10^{-5}$m) up to 0.0015 inches ($3.8x10^{-4}$m)) with the total thickness of the article of our invention including the backing layer, the medication reservoir lamina and the self adhesive layer being preferably about 3 mils.

Backing 10 in Figure 1 and backing 20 in Figure 2 substantially blocks loss of topical medicament from the plasticized PVC layer 11 in Figure 1 and 21 in Figure 2 other than in the direction of the surface which contacts self adhesive layer 12 in Figure 1 and 22 in Figure 2.

The self adhesive layer 12 in Figure 1 and 22 in Figure 2 is permeable to the topical medicament and may be continuous or discontinuous. After peeling away the peelable layer 13 from surface 14 in Figure 1, the article of Figure 1 may be adhered to that part of the skin which is to be subjected to treatment using the topical medicament located in lamina 11. The topical medicament in lamina 11 then is transported across junction 15 through the self adhesive layer 12 in Figure 1 or is transported from medication reservoir 21 across junction 24 through self adhesive layer 22 across surface 26 onto the skin.

The blended plasticized PVC which is coated onto the backing 10 in Figure 1 and backing 20 in Figure 2 can be fused into a homogeneous solid by heating it for a short period, such as 30 to 60 seconds at a temperature of, for example, 250 to 300°F (121 °C to 149 °C). The use of a plasticized PVC to form solid layer 11 in Figure 1 and solid layer 21 in Figure 2 enables layers 11 and 21 to be formed using a low temperature for a short period of time and provides conditions which do not affect the stability of the topical medicament.

When not in use, the entire surface intended for skin contact, e.g., surface 14 in Figure 1 and surface 24 in Figure 2 is preferably covered with a release layer, e.g., a release paper, such as release paper 13 in Figure 1 which is removed to expose the surface of adhesive layer 14 and topical medicament containing plasticized PVC layer 11 for application to the patient's skin.

The self adhesive layer 12 in Figure 1 and 22 in Figure 2 may be continuous or discontinuous as stated supra. In any case, the self adhesive may be any compatible self adhesive which will permit diffusion of the topical medicament therethrough (if, indeed, it is continuous) such as those set forth with specificity in Reissue Patent 31,886 issued on May 14, 1985

The example of a useful adhesive is that set forth in Example 3 at columns 12 and 13 of said Reissue Patent 31,886. Other self adhesives are set forth in U.S. Letters Patent 3,734,097.

The thickness of the self adhesive layer may vary from 0.5 mils ($1.3x10^{-5}$m) up to about 2 mils ($5.1x10^{-5}$m) with a preferred thickness of self adhesive layer being 1 mil ($2.5x10^{-5}$m).

The invention will now be described with reference to the following examples. In these examples where reference is made to moisture-vapor permeabilities, the units are grams/square meter/24 hours at 25°C at 75% relative humidity.

Example I

Oxygen and Water Vapor Permeation of the Medicated Wound Dressing Film Containing a Polyvinyl Pyrrolidone-Iodine Complex

The permeation of a gas or water vapor through a polymer film can be described in terms of the gas or water vapor dissolving at one surface of the film, diffusing through the film under a concentration gradient and evaporating from the other surface at the low concentration side.

If a constant pressure difference is maintained across the film, the gas vapor will diffuse through the film at a constant rate. Under steady state conditions, the permeability process is described by equation 1.

$$J = \frac{DS(p_1 - p_2)}{L} \qquad \text{(Eq. 1)}$$

Where:

J = the amount of gas or water vapor diffusing through unit area of the film in unit time at standard temperature and pressure.

D = the diffusion coefficient.

S = the solubility coefficient.

$p_1 - p_2$ = the pressure difference maintained across the film.

L = the film thickness.

The product D.S is defined as Permeability Constant (P) and is calculated from the equation 2.

$$P = DS = \frac{JL}{p_1 - p_2} \qquad \text{(Eq. 2)}$$

The permeability constant is expressed terms of quantities given below:

$$P = \frac{\text{(amount of gas or water vapor) x (thickness)}}{\text{(Area) x (Time) x (Pressure Difference)}}$$

Materials and Methods

The material tested for oxygen and water vapor permeation were the following:

1. A 1 mil thick Polyurethane film of a lower modulus, supplied by General Foam Corp.
2. A 1 mil thick film of Polyurethane blended with 10% of PVC supplied by Norwood Industries, Inc..
3. A 3 mil ($7.6x10^{-5}$m) thick PVC film of flesh color.
4. A 4.5 mil ($1.1x10^{-4}$m) thick medicated wound dressing Film (illustrated in Figure 1) containing an iodine complex and a surfactant (IGEPAL ® CO-630) in a dissolved form.
5. A 5 mil thick medicated wound dressing Film (illustrated in Figure 1) containing an iodine complex and a surfactant (IGEPAL ® CO-630) in a dissolved form.
6. A 5 mil ($3.2x10^{-4}$m) thick medicated wound dressing Film (illustrated in Figure 1) containing an iodine complex in a dispersed form.
7. A 1 mil ($5.1x10^{-5}$m) thick medicated wound dressing film manufactured by Minnesota Mining and Manufacturing company of St. Paul, Minnesota (TEGADERM ®).

The structure and composition characteristics of the wound dressing films Nos. 4,5 and 6 (which are embodiments of the invention) are presented in Figures 1 and 2 and Table 1, respectively. Films Nos. 1,2,3 and 7 are comparative.

The oxygen and water vapor permeabilities of the plain and wound dressing films were determined using the concentration increase method. The method employed a permeation cell which after being mounted with 5 $cm^2$ of film sample consisted of two different compartments. While the lower compartment was filled with nitrogen, the upper compartment was flushed continuously with oxygen humidified at a level of 65-75% (temperature = 25 ° C). The oxygen humidification was accomplished by passing an oxygen gas steam through a washing bottle filled with deionized water; The oxygen and water vapor from the upper compartment diffused through the tested film into lower compartment and changed its composition with time. The oxygen and water vapor concentration changes in the lower cell compartment were determined by chromatography. A research chromatograph (Hewlett Packard - 5750), was used for the chromatographic analysis. This chromatograph was equipped with a thermal conductivity detector and two 6'($1.5x10^{-1}$m) x 1/4" ($6x10^{-3}$m) O.D. aluminium column. Column #1 was packed with CTR1 and Column #2 with PORAPAK ® Q. The CTRI column was used for oxygen and the PORAPAK ® Q column for water vapor analysis. The GC operational conditions were as follows:

| | Oxygen Analysis | Water Vapor Analysis |
|---|---|---|
| Column temperature: | 25 °C | 150 °C |
| Detector temperature: | 150 °C | 250 °C |
| Injection port temperature: | 150 °C | 250 °C |
| Carrier gas: | Helium | Helium |
| Carrier gas flow rate: | 120cc/min | 50 cc/min |
| Filament current: | 150ma | 157ma |

Results and Discussion

The oxygen and water vapor permeability constants determined for the various wound dressing and other films are presented in Table 2. As it can be seen from this table, the oxygen permeability of the wound dressing films is not as high as that of the control. However, it can be considered sufficient enough to ensure permeation of the needed oxygen for wound healing. The high water vapor permeability constants of the same wound dressing films indicates that sweat or moisture can easily permeate through, which otherwise would collect on the inside surface of the film causing discomfort and possible adhesive failure.

Table 1: Composition of Medicated Wound Dressing Films

1. Medicatedwound dressing film containing an iodine complex in a dissolved form.

| | |
|---|---|
| Plasticizer: | 55% (ADMEX®) |
| PVC Resin: | 21% |
| PVP-Iodine Complex: | 8.6% |
| Ethanol: | 15.4% |

2. Medicated wound dressing film containing an iodine complex and a surfactant (IGEPAL® CO-630) in a dissolved form.

| | |
|---|---|
| Plasticizer: | 55% (ADMEX®) |
| PVC Resin: | 21% |
| PVP-Iodine Complex: | 8% |
| Surfactant: (IGEPAL® CO-630) | 1% |
| Ethanol: | 15% |

3. Medicated wound dressing film containing an iodine complex in a dispersed form.

| | |
|---|---|
| Plasticizer: | 65% (ADMEX®) |
| PVC Resin: | 25% |
| PVP-Iodine Complex: | 10% |

The curing conditions for the above wound dressing films were as follows:

| | |
|---|---|
| Temperature: | 300°F (149°C) |
| Curing Time: | 1 minute |

Table 2: Oxygen and Water Vapor Permeability Constants of Medicated Wound Dressing and Other Films

| Material | $O_2$ Permeability Constant at 25°C and 65% Relative Humidity (Per Unit Thickness) $\frac{(cm^3 O_2\ mil)}{(m^2 day\ atm.)}$ $\left(\frac{cm^3\ O_2 (2.54x10^{-5} m)}{m^2\ (8.64x10^4 s)\ atm}\right)$ | $O_2$ Permeability For Product of 3 Mils $\frac{(cm^3 O_2)}{(m^2 day\ atm.)}$ | Water Vapor Permeability Constant at 25°C and 75% Relative Humidity (Per Unit Thickness) $\frac{(g\ H_2O\ mil)}{(m^2 day\ atm)}$ | Water Vapor Permeability For Product of 3 Mils $(7.62x10^{-5}m)$ $\frac{(g\ H_2O)}{m^2 day\ atm.)}$ |
|---|---|---|---|---|
| Polyurethane Film | 4451 | - | 123.40 | - |
| Film of Polyurethane with 10% polyvinyl chloride | 2710 | - | 113.63 | - |
| Polyvinyl chloride film | 2226 | - | 22.04 | - |
| Wound Dressing Film with Polyvinylpyrrolidone-iodine complex dissolved | 6421.50 | 2140.50 | 379.10 | 126.30 |
| Wound Dressing with Polyvinylpyrrolidone-iodine complex and IGEPAL® CO-630 dissolved | 7725 | 2575 | 217.96 | 72.65 |

Table 2: Oxygen and Water Vapor Permeability Constants of Medicated Wound Dressing and Other Films

| Material | $O_2$ Permeability Constant at 25°C and 65% Relative Humidity (Per Unit Thickness) $\frac{cm^3 O_2 \text{ mil}}{m^2 \text{ day atm.}}$ $\left(\frac{cm^3\ O_2 (2.54x10^{-5}m)}{m^2\ (8.64x10^4 s)\ atm}\right)$ | $O_2$ Permeability For Product of 3 Mils $\frac{cm^3 O_2}{m^2 \text{ day atm.}}$ | Water Vapor Permeability Constant at 25°C and 75% Relative Humidity (Per Unit Thickness) $\frac{g\ H_2O \text{ mil}}{m^2 \text{ day atm}}$ | Water Vapor Permeability For Product of 3 Mils ($7.62x10^{-5}$m) $\frac{g\ H_2O}{m^2 \text{ day atm.}}$ |
|---|---|---|---|---|
| Wound Dressing Film with Polyvinylpyrrolidone-iodine complex dispersed | 7876.5 | 2625.5 | 151.80 | 50.60 |
| Wound Dressing Film Manufactured by Minnesota Mining and Manufacturing Co. of St. Paul, Minn. (TEGADERM®) | 7208.8 | 3604.4 (For Product of 2 mils ($5.1x10^{-5}$m) | 147.0 | 73.50 (For Product of 2 mils ($5.1x10^{-5}$m) |

NOTE: The polyurethane used is a polyurethane manufactured by Semex Medical Company, subsidiary of Seton Company of Malverne, Pennsylvania. It is identified according to the infrared spectrum of Figure 6.

Example II

Mechanical Properties of Some Polymer Films

The following polymer films were tested for their ultimate tensile strength, elongation at break and Young's Modulus and the results are as follows:

Table 3: Mechanical Properties of Some Polymer Films

| Sample | Thickness (Mils) ($2.5x10^{-5}$ m) | Initial Length (In ($2.5 x10^{-2}$ m)) | Width (In ($2.5 x 10^{-2}$ m)) | Strain Rate (In/Min ($4.2x10^{-4}$ m/s)) | Peak Load (PDS 0.45 kg) | Ultimate Tensile (PDS/In$^2$ (9 $x10^2$ Kg/m$^2$)) | Break Extension (In) ($2.5x10^{-2}$ m) | Elongation (%) | Young's Modulus |
|---|---|---|---|---|---|---|---|---|---|
| Polyvinyl-chloride Film | 3 | 8 | 1 | 12 | 4,78 | 3187 | 7,03 | 87,84 | 8514±464 |
| Polyurethane Film with a Lower Modulus | 1 | 3 | 0,5 | 12 | 2,98 | 5960 | 13,75 | 458,3 | 1480±56 |
| Polyurethane Film Containing 5% Polyvinylchloride | 1 | 8 | 0,5 | 12 | 1,58 | 3153 | 21,72 | 271.5 | 3158±0 |
| Polyurethane Film Containing 10% Polyvinyl chloride (First Sample) | 1 | 8 | 0,5 | 12 | 1.67 | 3340 | 14,36 | 179,50 | 8070±604 |
| Polyurethane Film Containing 10% Polyvinylchloride (Second Sample) | 1 | 8 | 1 | 12 | 3,10 | 3101 | 15,28 | 190,96 | 11236 |

Figure 3 sets forth a graph of stress relaxation of various materials versus time. The graph indicated by reference numeral 30 is the graph for polyurethane film which is the polyester polyurethane identified according to the infrared spectrum of Figure 6. The graph indicated by reference numeral 31 is the graph for the polyurethane resin also containing 5% polyvinyl chloride. The graph indicated by reference numeral 32 is a graph for the polyurethane resin containing 10% by weight of polyvinyl chloride. The graph

indicated by reference numeral 33 is a graph for polyvinyl chloride per se (time versus stress relaxation) (percent).

Example III

Antibacterial Activity Evaluation of the Wound Dressing Film Containing Polyvinylpyrrolidone-iodine Complex

Objective

The objective of this study was to find out if the wound dressing film could release sufficient amount of iodine necessary to maintain the microenvironment of a wound free of bateria.

Materials and Methods

The medicated wound dressing films submitted for antibacterial activity determination were the following:

1. A wound dressing film containing 11.2% of polyvinylpyrrolidone-iodine complex;
2. A wound dressing film containing 24% of polyvinylpyrrolidone-iodine complex;

A porous pressure sensitive adhesive, supplied by Norwood Industries, Inc., was used as face adhesive on both films (#1 and #2).

3. A wound dressing film containing 24% of iodine complex. The adhesive used with this sample was the #408 supplied by the Minnesota Mining and Manufacturing Company.
4. Control - The IOBAN ® 2-6635 antimicrobial film of Minnesota Mining and Manufacturing Company of St. Paul, Minnesota.

The composition and structure characteristics of the wound dressing films are presented in Table 4 and Figures 1 and 2.

The antibacterial activity of the tested samples was determined by using the AATCC Method 90. Staphylococcus aueeus and Escherichia coli cultures were used to evaluate the product's efficiency on human skin, and its activity in preventing the spread of enteric disease. The incubation period was 24 hours ($8.64 \times 10^4$ s) at 37 °C.

Results and Discussion

The results of the antibacterial activity evaluation obtained for the tested wound films are presented in Table 5. As can be seen from Table 5, the wound dressing films of our invention performed as well as the control. There was no zone of inhibition surrounding the samples. Bacterial growth did not occur, only where there was direct contact between the wound dressing films and the culture medium.

Table 4

| Composition of the Wound Dressing Films Submitted for Antibacterial Activity Determination | |
|---|---|
| Wound Dressing #1 | |
| Polyvinylpyrrolidone-iodine complex: | 11.2% |
| ADMEX ® Plasticizer: | 49.6% |
| PVC Resin (6338): | 37.8% |
| Polyvinylpyrrolidone: | 1.4% |
| Wound Dressing Films #2 and #3 | |
| Polyvinylpyrrolidone-iodine complex: | 24% |
| ADMEX ® Plasticizer: | 42% |
| Polyvinyl chloride resin (6338) | 29.5% |
| Polyvinylpyrrolidone (molecular weight 40,000): | 4.5% |

Table 5

| Results of the Antibacterial Activity Evaluation | | |
|---|---|---|
| Sample Identification | Staphylococcus Aureus | Escherichia Coli |
| Sample with 11.2% of polyvinylpyrrolidoneiodine complex and a porous face adhesive | No growth | No growth |
| Sample with 24% of polyvinylpyrrolidoneiodine complex and a porous face adhesive | No growth | No growth |
| Sample with 24% of polyvinylpyrrolidoneiodine complex and Minnesota Mining and Manufacturing Company #408 face adhesive | No growth | No growth |
| Control (IOBAN ®2) | No growth | No growth |

Example IV

Hydrocortisone Film Dressing

A. In Vitro Hydrocortisone Release Testing

The release of hydrocortisone from the film dressing of our invention was studied using Franz diffusion cells employing 20% aqueous propylene glycol as the receptor phase. Samples of the receptor phase were taken at different time intervals and analyzed for hydrocortisone using ultraviolet spectroscopy. The release profile (Figure 4 (indicated by reference numeral 40 with the axis for time in hours indicated by reference numeral 42 and the axis for fraction release indicated by reference numeral 41)) indicated that 25% and 75% of the total hydrocortisone in the sample was released within periods of 4 hours and 24 hours, respectively.

The in vitro data illustrates the sustained hydrocortisone release capability of the film dressing of our invention. In in vivo performance on skin, the sustained release of hydrocortisone would be expected to maintain its saturation concentration at the skin surface.

B. Product Indications and Advantages

For antiinflammatory action in the treatment of pruritic, allergic and atopic dermatitis. As compared to an ointment, the film dressing is more convenient to use, because it is non-greasy and is not wiped off by clothes or washed away by water. Permits greater freedom of movement to the patient. Longer lasting action. Breathability and skin-like appearance and wear can enhance patient acceptance.

Experimental

A. Materials

Acetone and isopropyl alcohol, supplied by VWR Scientific Propylene glycol, supplied by Fisher Scientific Hydrocortisone, supplied by Sigma Chemical Company Plasticizer (ADMEX ® 760), supplied by Nuodex, Inc.

PVC resin 6338, supplied by Occidental Co. Gelva 737 adhesive, supplied by Monsanto.

Film of polyurethane blended with 10% PVC, supplied by Norwood Industries, Inc.

Aerosil 200 (silica powder) supplied by Degussa Inc.

B. Methods

Dissolution Study

This study was designed to determine the release of hydrocortisone from the dispenser of Figure 1 as affected by the amount of active drug (hydrocortisone), plasticizer (ADMEX ®) and PVC resin in the reservoir. In addition, the effect of various face adhesives on the transport and release of hydrocortisone was evaluated.

Construction of the Hydrocortisone Dispenser

All formulation ingredients were dissolved in a solvent (isopropyl Alcohol/Acetone: 2/1) and mixed thoroughly to get a suspension. Furthermore, this suspension was cast on a release liner, using a Gardner knife, to get the reservoir preferably 1 mil thick. The reservoir was stripped of the residual solvent by placing it for

30 minutes in an oven set at 90°C. The PVC-polyurethane backing film was then laminated to one side of the reservoir by simutaneously passing both of them through a hot nip laminator. The operating conditions for the laminator were as follows:

| | |
|---|---|
| Temperature: | 300°F (149°C) |
| Pressure: | 40 PSI (1.9x$10^3$ N/$m^2$) |
| RPM: | 7 |

The construction of the hydrocortisone dispenser was completed by transferring a pressure sensitive adhesive on the other side of the reservoir after the release liner was removed.

Receiver Solution Preparation

The receiver solution used in this study was deionized water containing 20% of propylene glycol.

Cell Preparation

The Franz type diffusion cells, 0.732 $cm^2$, were used for this study. The receiver compartments were filled with 5 ml of receiver solution and one small magnetic stir bar was added to each cell. Donor reservoirs were 3/16" (4.7x$10^{-2}$m) disks which had been punched out of a hydrocortisone dispenser. The disks were mounted on the cell and covered with aluminum foil to assure proper disk position. The top of the cell was clamped in place and the entire cell was put in a heated block (33-34°C). Three replicates were run.

Sampling Procedure

Samples of the receiver solution were taken at designated time intervals. The entire receiver was sampled and replaced by 5 ml of fresh solution. The samples were analyzed immediately thereafter.

Sampling Analysis

Samples were analyzed for hydrocortisone, using a Perkin Elmer UV spectrophotometer set at a wave length of 247 nm. A calibration curve was used to convert absorbance values to concentration.
The cumulative hydrocortisone concentrations were then plotted as a function of time. The slopes of the lines were calculated using a regression analysis and averaged. The slope represents the release rate of hydrocortisone, (micrograms/$cm^2$-hr.).

Examples V - IX, Inclusive

In the following Examples V - IX, various topical medicaments were added in the percents indicated to the polyvinyl chloride plastisol layer and amount of drug released was measured against time, In each case, the backing film was 1 mil thick; the adhesive layer was 1 mil thick and was composed of acrylic pressure sensitive adhesive. The backing layer was 10% polyvinyl chloride and 90% polyurethane-polyester which polyurethane-polyesters defined according to the infrared spectrum of Figure 6 and was manufactured by the Semex Medical Subsidiary of Seton Company of Malverne, Pennsylvania. In each case, the plasticizer used is ADMEX ® defined according to the infrared spectrum of Figure 5.

Example V

Tolnaftate Film Dressing

A film dressing according to Figure 1 was prepared wherein in the medicament reservoir layer, the following composition was prepared:

| | |
|---|---|
| ADMEX ® plasticizer | 56% |
| Polyvinyl chloride | 43% |
| Tolnaftate | 1% |

The percent of drug released over a period of time was as follows:

| Time | Percent Drug Released |
|---|---|
| 5 hours | 20% |
| 10 hours | 30% |
| 24 hours | 45% |
| 48 hours | 78% |

Example VI

Chlorhexidine Diacetate Hydrate Film Dressing

A film dressing according to Figure 1 was prepared wherein the medicament polyvinyl chloride plastisol reservoir lamina was composed of the following composition:

| | |
|---|---|
| Polyvinyl chloride | 41% |
| ADMEX ® plasticizer | 55% |
| Chlorhexidine diacetate hydrate | 4% |

The time versus percent drug release data is as follows:

| Time | Percent Drug Released |
|---|---|
| 7 hours | 6.5% |
| 24 hours | 10.4% |
| 48 hours | 13.0% |

In vitro antimicrobial activity testing for this product showed a 1-2 mm wide zone of inhibition against Staphylococcus aureus and Escherichia coli bacteria.

Example VII

Hydrocortisone Film Dressing

A hydrocortisone film dressing was prepared according to the structure of Figure 1 wherein the polyvinyl chloride plastisol medicament reservoir lamina contained the following ingredients:

| | |
|---|---|
| Polyvinyl chloride | 42.5% |
| ADMEX ® plasticizer | 57.0% |
| Hydrocortisone | 0.5% |

The percent drug released versus time is as follows:

| Time | Percent Drug Released |
|---|---|
| 4 hours | 20% |
| 8 hours | 34% |
| 24 hours | 70% |

Example VIII

Benzalkonium Chloride Film Dressing

A film dressing according to the construction of Figure 1 was prepared wherein the polyvinyl chloride plastisol medicament reservoir lamina consisting of the following ingredients:

| | |
|---|---|
| Polyvinyl chloride | 48.8% |
| ADMEX ® plasticizer | 51.0% |
| Benzalkonium chloride | 0.2% |

Example IX

Benzethonium Chloride Film Dressing

A film dressing according to the construction of Figure 1 was prepared wherein the polyvinyl chloride plastisol medicament reservoir lamina consisted of the following ingredients:

| | |
|---|---|
| Polyvinyl chloride | 48.8% |
| ADMEX ® plasticizer | 51.0% |
| Benzethonium chloride | 0.2% |

## Claims

**1.** A moisture-vapor permeable and oxygen-permeable adhesive dressing for use on human skin which dressing is unaffected by and impermeable to water in the liquid phase, which dressing when in use on human skin consists essentially of:

(i) a polymeric backing material lamina (20) composed of a mixture of polyurethane and polyvinyl chloride and having a first substantially planar surface (23) and a second substantially planar surface (25);

(ii) adhering to said first planar surface (23) of said backing material, a medication reservoir lamina (21) having a first substantially planar surface and a second substantially planar surface (24) consisting essentially of an intimate mixture of:

(a) a polyvinyl chloride polymer;

(b) a polymeric plasticizer intimately admixed with said polyvinyl chloride and compatible with said polyvinyl chloride; and;

(c) a topical medicament compatible with said polyvinyl chloride and said plasticizer;

said first substantially planar surface of said medication reservoir lamina adhering to said first substantially planar surface of said backing material in a continuous or discontinuous manner; and

(iii) the second substantially planar surface (24) of said medication reservoir lamina being adhered to pressure-sensitive adhesive (22) which is permeable to oxygen and moisture vapour but is unaffected by liquid water;

(iv) the moisture-vapor-permeability of the dressing being at least 25 g/sq. meter/24 hours at 25 °C at 75% relative humidity.

**2.** The dressing of claim 1 wherein the pressure-sensitive adhesive (22) comprises an acrylic ester copolymer containing hydrophilic groups wherein the hydrophilic group is hydroxyl, carboxyl, amine,

amide, ether or alkoxy, said adhesive adhering continuously to said medication reservoir lamina (21).

3. The dressing of claim 1 or claim 2 wherein the backing material lamina (20) is thermoplastic and consists of an intimate mixture of polyurethane resin and polyvinyl chloride, the weight percent of polyvinyl chloride in said first intimate mixture being from about 5% up to about 15%.

4. The dressing of any one of claims 1 to 3 wherein the moisture-vapor-permeability thereof is at least 40 g/sq. meter/24 hours at 25 °C at 75% relative humidity.

5. The dressing of any one of claims 1 to 4 wherein the topical medicament is selected from the group consisting of:
   (i) tolnaftate;
   (ii) chlorhexidine;
   (iii) chlorhexidine esters;
   (iv) chlorhexidine salts;
   (v) hydrocortisone;
   (vi) benzalkonium salts;
   (vii) benzethonium salts; and
   (viii) polyvinylpyrrolidone-iodine complexes.

6. The dressing of any one of claims 1 to 5 wherein the plasticizer has the infrared spectrum of Figure 5.

**Patentansprüche**

1. Wasserdampf- und sauerstoffdurchlässiger haftender Verband zur Verwendung auf menschlicher Haut, wobei der Verband von Wasser in der Flüssigphase nicht beeinträchtigt wird und dafür undurchlässig ist und wobei der Verband bei Gebrauch auf menschlicher Haut im wesentlichen aus folgendem besteht:
   (i) einer polymeren Trägermaterialschicht (20), bestehend aus einem Gemisch aus Polyurethan und Polyvinylchlorid, mit einer ersten im wesentlichen ebenen Oberfläche (23) und einer zweiten im wesentlichen ebenen Oberfläche (25);
   (ii) einer an der ersten ebenen Oberfläche (23) des Trägermaterials haftenden Arzneistoffreservoirschicht (21), die eine erste im wesentlichen ebene Oberfläche und eine zweite im wesentlichen ebene Oberfläche (24) hat und im wesentlichen aus einem innigen Gemisch von folgendem besteht:
      (a) einem Polyvinylchloridpolymer;
      (b) einem polymeren Weichmacher, der mit dem Polyvinylchlorid innig vermischt und mit dem Polyvinylchlorid kompatibel ist; und
      (c) einem topikalen Arzneistoff, der mit dem Polyvinylchlorid und dem Weichmacher kompatibel ist;

   wobei die erste im wesentlichen ebene Oberfläche der Arzneistoffreservoirschicht an der ersten im wesentlichen ebenen Oberfläche des Trägermaterials auf kontinuierliche oder diskontinuierliche Weise haftet; und
   (iii) wobei die zweite im wesentlichen ebene Oberfläche (24) der Arzneistoffreservoirschicht an Kontaktklebstoff (22) haftet, der für Sauerstoff und Wasserdampf durchlässig ist, aber von flüssigem Wasser nicht beeinträchtigt wird;
   (iv) wobei die Wasserdampfdurchlässigkeit des Verbands mindestens 25 $g/m^2/24$ h bei 25 °C und 75 % relativer Feuchte ist.

2. Verband nach Anspruch 1, wobei der Kontaktklebstoff (22) ein Acrylsäureester-Copolymer aufweist, das hydrophile Gruppen enthält, wobei die hydrophile Gruppe Hydroxyl, Carboxyl, Amin, Amid, Ether oder Alkoxy ist, wobei der Klebstoff kontinuierlich an der Arzneistoffreservoirschicht (21) haftet.

3. Verband nach Anspruch 1 oder 2, wobei die Trägermaterialschicht (20) thermoplastisch ist und aus einem innigen Gemisch aus Polyurethanharz und Polyvinylchlorid besteht, wobei der Gewichtsprozentanteil von Polyvinylchlorid in dem ersten innigen Gemisch ca. 5 % bis ca. 15 % beträgt.

4. Verfahren nach einem der Ansprüche 1-3, wobei seine Wasserdampfdurchlässigkeit mindestens 40 $g/m^2/24$ h bei 25 °C und 75 % relativer Feuchte ist.

5. Verband nach einem der Ansprüche 1-4, wobei der topikale Arzneistoff aus der Gruppe gewählt ist, die besteht aus:
   (i) Tolnaftat;
   (ii) Chlorhexidin;
   (iii) Chlorhexidinestern;
   (iv) Chlorhexidinsalzen;
   (v) Hydrocortison;
   (vi) Benzalkoniumsalzen;
   (vii) Benzethoniumsalzen; und
   (viii) Polyvinylpyrrolidon-Iod-Komplexen.

6. Verband nach einem der Ansprüche 1-5, wobei der Weichmacher das IR-Spektrum von Figur 5 hat.

**Revendications**

1. Pansement adhésif perméable à la vapeur d'eau et perméable à l'oxygène, destiné à être utilisé sur la peau humaine, lequel pansement n'est pas affecté par l'eau en phase liquide et imperméable à celle-ci, lequel pansement, lorsqu'il est utilisé sur la peau humaine, est essentiellement constitué de :
   (i) un feuillet en une matière de renforcement polymère (20) composée d'un mélange de polyuréthane et de chlorure de polyvinyle et ayant une première surface sensiblement plane (23) et une seconde surface sensiblement plane (25) ;
   (ii) adhérant à ladite première surface plane (23) de ladite matière de renforcement, un feuillet de réservoir à médicament (21) avant une première surface sensiblement plane et une seconde surface sensiblement plane (24) essentiellement constitué d'un mélange intime de :
      a) un polymère de chlorure de polyvinyle ;
      b) un plastifiant polymère en mélange intime avec ledit chlorure de polyvinyle et compatible avec ledit chlorure de polyvinyle ; et
      c) un médicament topique compatible avec ledit chlorure de polyvinyle et ledit plastifiant ;
   ladite première surface sensiblement plane dudit feuillet de réservoir à médicament adhérant à ladite première surface sensiblement plane de ladite matière de renforcement d'une manière continue ou discontinue ; et
   (iii) la seconde surface sensiblement plane (24) dudit feuillet de réservoir à médicament étant collée à un adhésif sensible à la pression (22) qui est perméable à l'oxygène et à la vapeur d'eau mais n'est pas affecté par l'eau liquide ;
   (iv) la perméabilité à la vapeur d'eau du pansement étant d'au moins 25 $g/m^2/24h$ à 25 °C et 75% d'humidité relative.

2. Pansement selon la revendication 1, dans lequel l'adhésif sensible à la pression (22) comprend un copolymère d'ester acrylique contenant des groupes hydrophiles dans lequel le groupe hydrophile est de l'hydroxyle, carboxyle, amine, amide, éther ou alcoxy, ledit adhésif adhérant de manière continue audit feuillet de réservoir à médicament (21).

3. Pansement selon la revendication 1 ou la revendication 2, dans lequel le feuillet en matière de renforcement (20) est thermoplastique et consiste en un mélange intime de résine de polyuréthane et de chlorure de polyvinyle, le pourcentage en poids de chlorure de polyvinyle dans ledit premier mélange intime étant d'environ 5% à environ 15%.

4. Pansement selon l'une quelconque des revendications 1 à 3, dans lequel la perméabilité à la vapeur d'eau de celui-ci est d'au moins 40 $g/m^2/24h$ à 25 °C et 75% d'humidité relative.

5. Pansement selon l'une quelconque des revendications 1 à 4, dans lequel le médicament topique est choisi dans le groupe constitué de :
   (i) tolnaftate ;
   (ii) chlorhexidine ;
   (iii) esters de chlorhexidine :
   (iv) sels de chlorhexidine ;
   (v) hydrocortisone ;
   (vi) sels de benzalkonium ;

(vii) sels de benzéthonium ; et
(viii) complexes polyvinylpyrrolidone-iode.

**6.** Pansement selon l'une quelconque des revendications 1 à 5, dans lequel le plastifiant a le spectre infra-rouge de la figure 5.

FIG.1

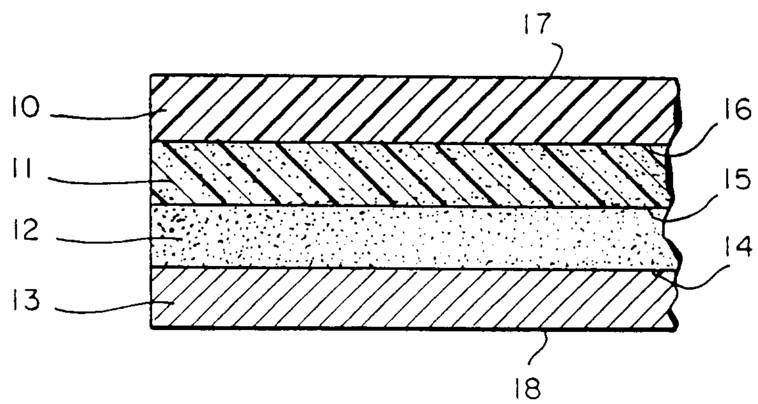


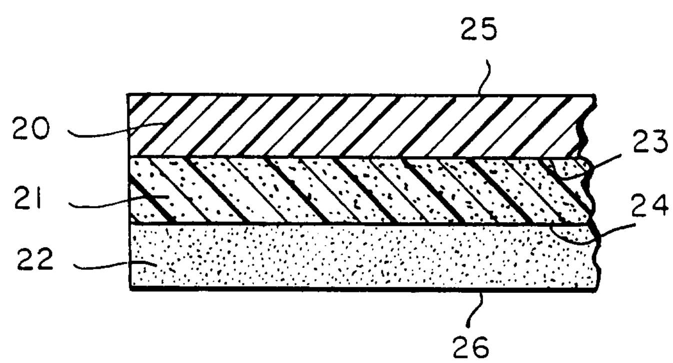


FIG.2

FIG.3

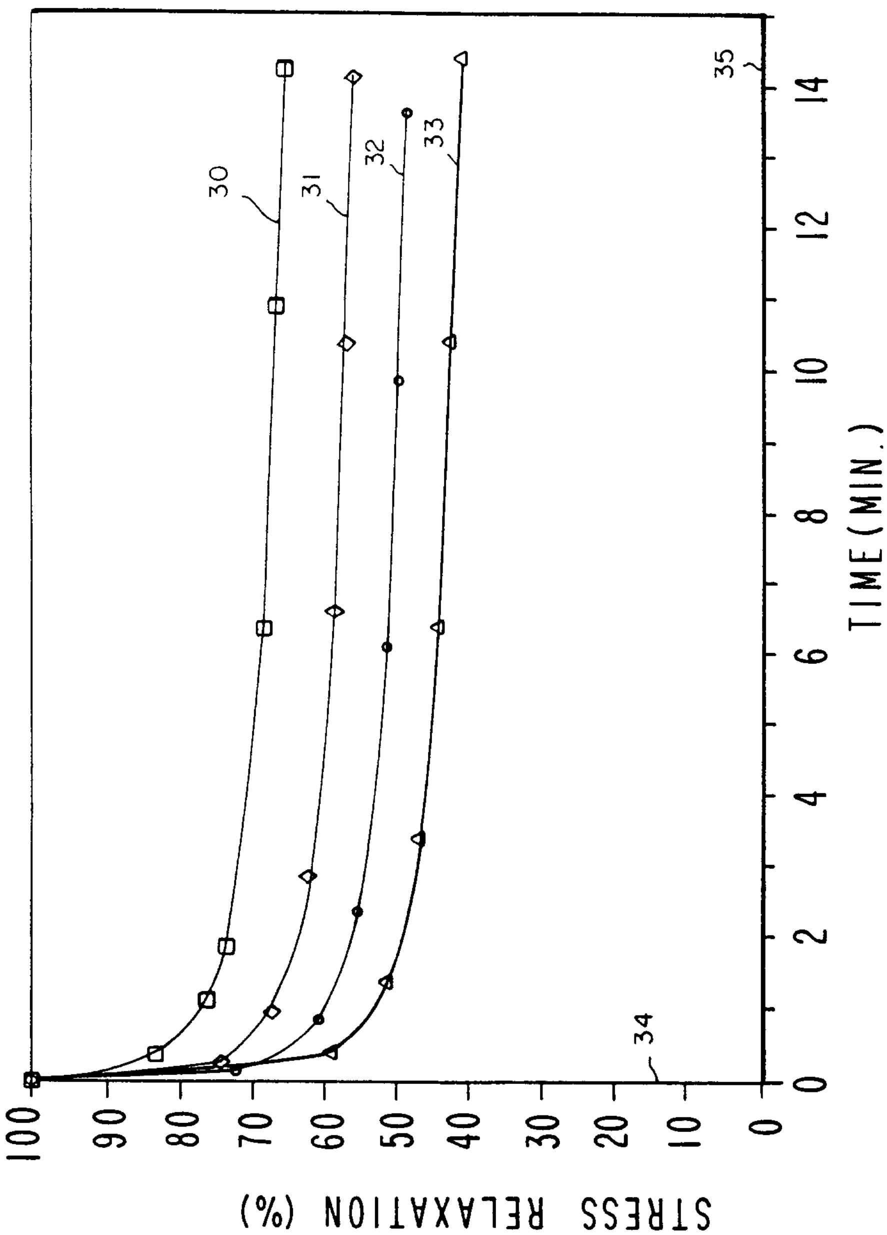
STRESS RELAXATION (%)
100
90
80
70
60
50
40
30
20
10
0
TIME (MIN.)
0
2
4
6
8
10
12
14
30
31
32
33
34
35

FIG.4

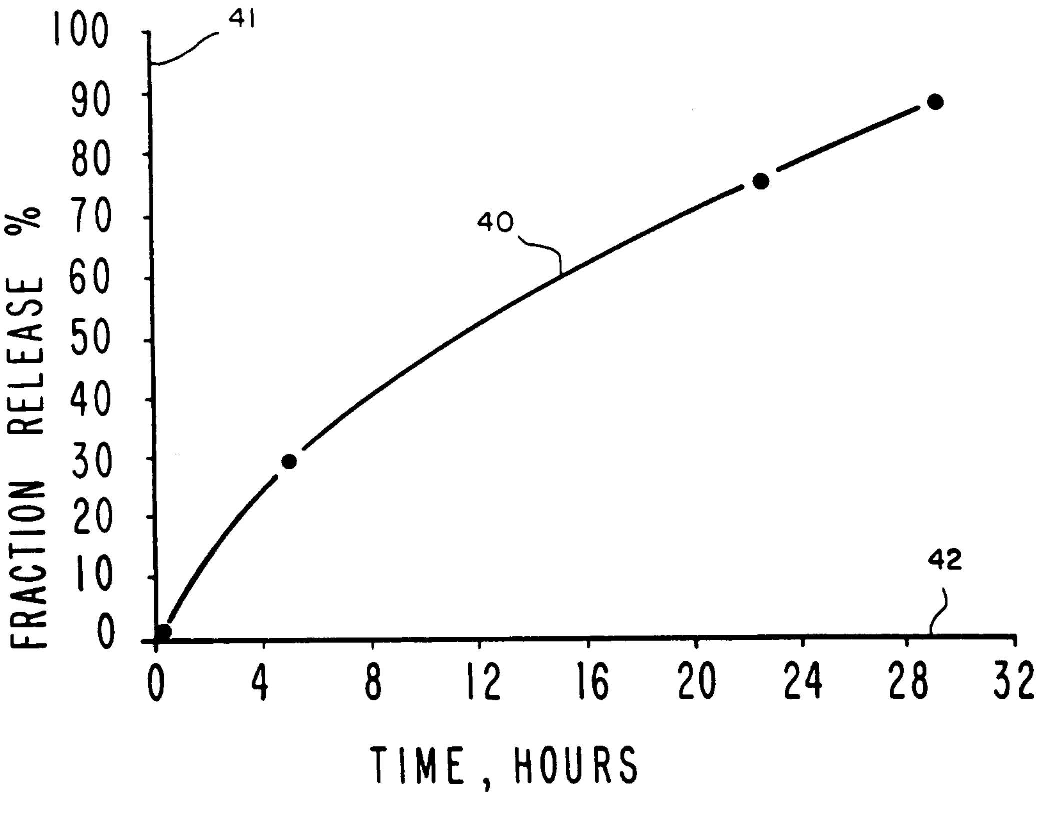
100
90
80
70
60
50
40
30
20
10
0
FRACTION RELEASE %
41
40
42
0
4
8
12
16
20
24
28
32
TIME, HOURS

FIG.5

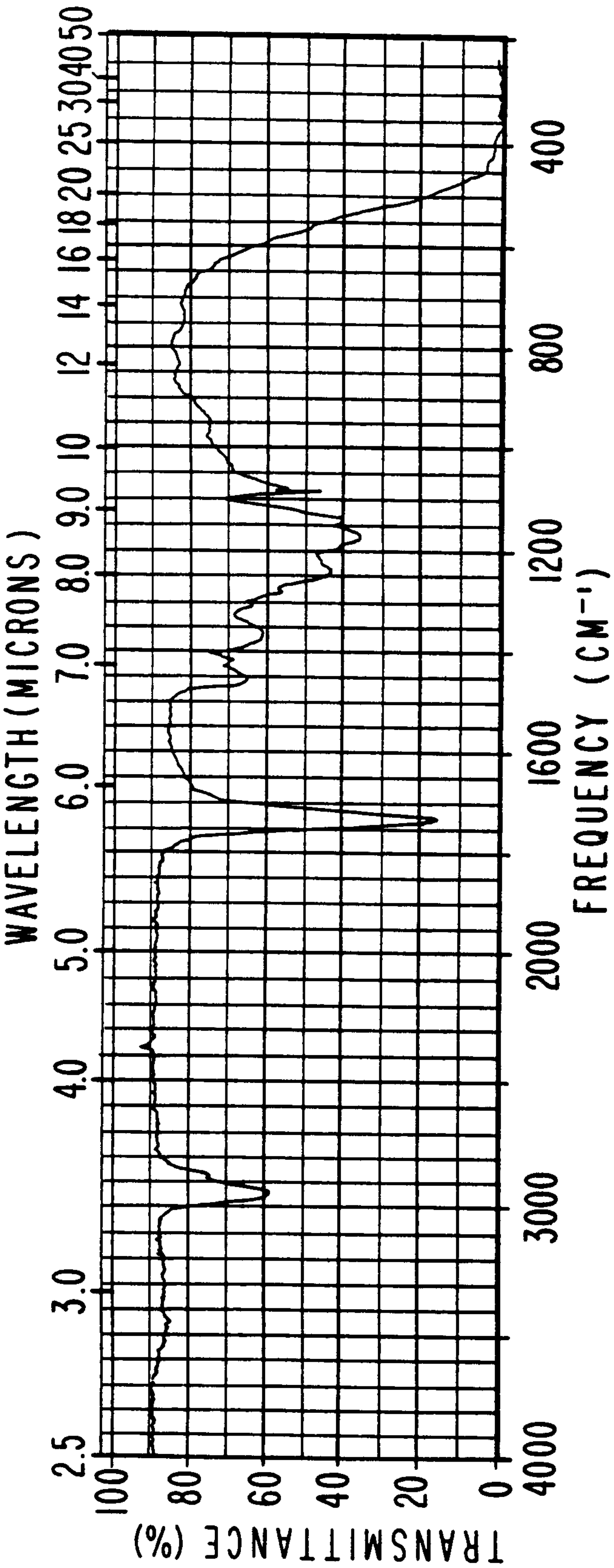


I R SPECTRUM

FIG.6

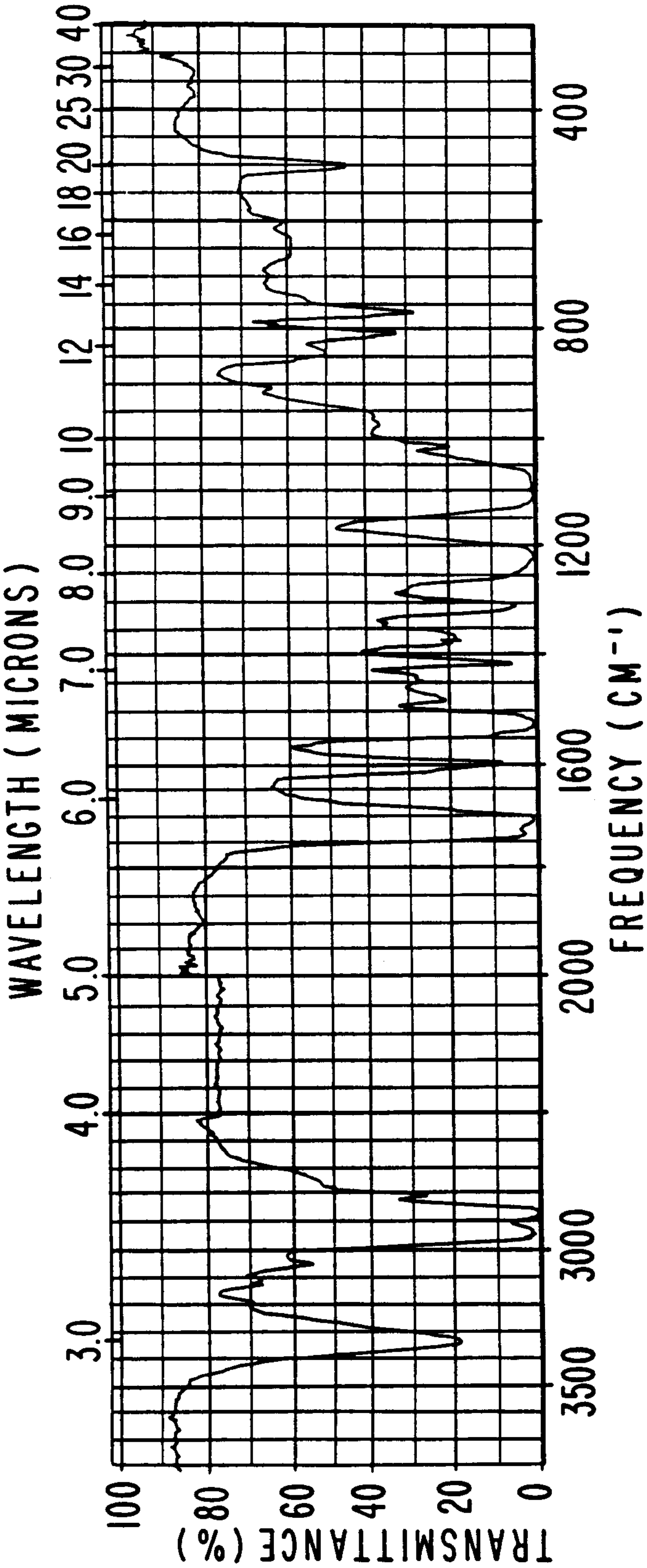


INFRARED SPECTRUM